# NEUE EUROPÄISCHE PATENTSCHRIFT

(11) **EP 1 021 473 B2**
(45) Veröffentlichungstag und Bekanntmachung des Hinweises auf die Entscheidung über den Einspruch: **01.12.2004**
(45) Hinweis auf die Patenterteilung: 09.01.2002
(21) Anmeldenummer: 98958794.4
(22) Anmeldetag: 24.09.1998
(51) Int. Cl.: C08G 18/20

(54) **BIS(ALKYLAMINO)ALKYLETHER-VERBINDUNGEN UND IHRE VERWENDUNG**
BIS(ALKYLAMINO)ALKYLETHER COMPOUNDS AND THE USE THEREOF
COMPOSES DE BIS(ALKYLAMINO)ALKYLETHER ET LEUR UTILISATION

(30) Priorität: 26.09.1997 DE 19742720
(43) Veröffentlichungstag der Anmeldung: 26.07.2000
(73) Patentinhaber: Performance Chemicals Handels GmbH, 21244 Buchholz (DE)
(72) Erfinder: KLOCKEMANN, Werner, D-21244 Buchholz (DE); SIKORSKI, Matthias, D-22117 Hamburg (DE); BADER, Michael, Roland, D-23843 Bad Oldesloe (DE)
(74) Vertreter: Schupfner, Georg U.
(86) Internationale Anmeldenummer: PCT/DE1998/002897
(87) Internationale Veröffentlichungsnummer: WO 1999/016807

(56) Entgegenhaltungen:
- EP-A- 0 812 866
- DE-C- 841 917
- US-A- 3 645 925
- US-A- 4 048 107
- US-A- 4 333 680
- US-A- 4 780 520
- US-A- 5 086 151
- US-A- 5 195 946
- DATABASE CHEMABS CHEMICAL ABSTRACTS SERVICE, COLUMBUS, OHIO, US an: 97:216103, XP002092863 & MOVSUMZADE M.M. ET AL.: AZERB. KHIM. ZH., Nr. 3, 1982, Seiten 30-34,

## Beschreibung

Gegenstand der Erfindung sind Bis(alkylamino)alkylether-Verbindungen, Zusammensetzungen enthaltend Bis(alkylamino)alkylether-Verbindungen und Polyurethane- und/oder Polyharnstoff-Verbindungen sowie die Verwendung der Bis(alkylamino)alkylether-Verbindungen zur Herstellung von Polyurethan-und/oder Polyharnstoff-Schaumstoffen.

Als Polyurethane werden Polymere mit sehr unterschiedlicher Zusammensetzung bezeichnet. Allen Polyurethanen ist gemeinsam, daß sie nach dem Diisocyanat-Polyadditionsverfahren hergestellt werden und als charakteristisches Kettenglied Urethan-Gruppen aufweisen. Der Anteil der Urethan-Gruppen gegenüber anderen die Monomereinheiten verknüpfenden Gruppen kann jedoch von untergeordneter Bedeutung sein. Neben der Urethan-Bindung können unter anderem Harnstoff-, Amid-, Biuret- und Isocyanurat-Bindungen durch die Isocyanat-Reaktion ausgebildet werden.

In vielen industriell bedeutenden Polyurethan-Kunststoffen verknüpfen die Urethangruppen Polyalkylenetheroder Polyestersequenzen, die ihrerseits Molekulargewichte von 200 bis 6000 g/mol aufweisen können.

Bedingt ist die Vielfalt möglicher Verknüpfungen nicht nur durch die Reaktivität der Isocyanat-Bindung, sondern auch durch die Vielzahl unterschiedlicher gegenüber der Isocyanat-Gruppe reaktionsfähige Verbindungen. Weiterhin können hinsichtlich ihrer Funktionalität unterschiedliche. Verbindungen gleichzeitig, nebeneinander eingesetzt werden. Die gebräuchlichsten Verbindungen neben den Diisocyanaten weisen Alkohol-, Carbonsäure- und/oder Amin-Gruppen auf. Daneben spielt aber auch die Reaktion der Isocyanat-Gruppe mit Wasser eine wichtige Rolle.

Die Reaktionen der NCO-Gruppe sind ganz außerordentlich empfindlich gegenüber Katalysatoren der verschiedensten Art. Sowohl Lewis-Basen als auch Lewis-Säuren sind wirksame Katalysatoren. Die wichtigsten Lewis-Basen sind tertiäre Amine unterschiedlichster Struktur, z.B. Diaza-bicyclo-octan, Triethylamin, Dimethylbenzylamin, Bis-dimethylaminoethylether und Dimethyl ethanolamin.

Die wichtigsten katalytisch wirkenden Lewis-Säuren sind zinnorganische Verbindungen, wie Zinn-diethylhexoat, Dibutylzinn-dilaurat, Dibutylzinn-bis-dodecyl-mercaptid, und Blei-phenyl-ethyl-dithiocarbaminat.

Die Katalysatoren werden oft als System, d.h. als Kombination mehrerer Katalysatoren, etwa einer LewisBase mit einer Lewis-Säure, eingesetzt. Der Katalysator/das Katalysator-System wird zur Beschleunigung von zwei manchmal drei Hauptreaktionen eingesetzt, die gleichzeitig und oft in Konkurrenz zueinander ablaufen.

Eine dieser Reaktionen ist die kettenverlängernde Isocyanat-Hydroxyl-Reaktion, bei der ein hydroxylhaltiges Molekül mit einem isocyanathaltigem Molekül unter Bildung eines Urethans reagiert. Bei dieser Reaktion wird ein sekundäres Stickstoffatom in den Urethangruppen erzeugt. Sind neben den Hydroxylgruppen auch noch andere reaktiven Wasserstoff aufweisende Gruppen, wie etwa Amin-Gruppen, anwesend, tragen auch andere chemische Verknüpfungen (z.B. Harnstoff-Gruppen) zum Kettenaufbau bei.

Die zweite Reaktion ist die Isocyanat-Wasser-Reaktion, bei der ein Molekül mit Isocyanat-Endgruppe unter Bildung einer Harnstoff-Gruppe verlängert wird und Kohlendioxid erzeugt. Das freiwerdende Gas wirkt als Treibmittel, um den Schaum zu treiben oder das Expandieren des Schaums zu unterstützen. Durch die Isocyanat-Wasser-Reaktion wird in-situ entweder das gesamte Gas oder auch nur ein Teil davon für die Schaumreaktion erzeugt.

Die Reaktionen müssen gleichzeitig mit optimal aufeinander abgestimmter Geschwindigkeit ablaufen, damit eine gute Schaumstruktur erhalten wird. Wenn die Kohlendioxidentwicklung im Vergleich zur Kettenverlängerung zu rasch verläuft, fällt der Schaum zusammen. Wenn die Kettenverlängerung im Vergleich zur Kohlendioxidentwicklung zu rasch erfolgt, wird die Steighöhe des Schaums begrenzt, wodurch ein Schaum hoher Dichte mit einem hohen prozentualen Anteil an schlecht ausgebildeten Zellen entsteht. Bei Fehlen einer ausreichenden Vernetzung ist der Schaum nicht stabil.

Es ist bekannt, daß tertiäre Amine die Vernetzungsreaktion wirksam katalysieren. Einige der tertiären Amine, wie z.B. Bisdimethylaminoethylether, sind auch wirksame Katalysatoren der Wasser-Isocyanat-Reaktion. Bismorpholinoethylether sind hierfür vorgeschlagen worden (US 3,661,808). Diese weisen jedoch eine zu schnelle Hautbildung zu Beginn der Reaktion auf. Durch diese geschlossene Haut wird ein Eindringen der für die Reaktion benötigten Feuchtigkeit erschwert, so daß die Aushärtung des Polymers im Kern nur langsam abläuft. Insbesondere bei Montageschäuman macht sich diese Nachreaktion, die ein Nachdrücken des Schaumes zur Folge hat, störend bemerkbar. Ähnliches gilt für die aus der WO 93/01178-A1 bekannten Polyurethankatalysatoren: 2-(N-3-methylpiperidino)-2'-(N-Hexamethylenimino) diethylether, 2,2'-Bis(N,N'-bishexamethylenimino) diethylether, 2-(N-(3-Methyl)plperidino)-2'-(N-Hexarnethylenimino) diethylether und 2-N-Morpholino-2'-N-pyrrolidino diethylether.

in der DE 43 27 498 wird α-Morpholino-ethoxy-ethoxy-β-morpholino-ethan als Polyurethan-Kataysator zur Herstellung von orthopädische Polyurethan-Verbandsmaterial offenbart. Der Polyurethan-Kunststoff hat hier ausschließlich die Funktion eines Klebstoffes.

Es ist die Aufgabe der vorliegenden Erfindung, einen Katalysator bereitzustellen, der selektiv in Bezug auf die Förderung der Isocyanat-Wasser-Reaktion ist und zur Katalyse der Kettenverlängerungsreaktion inaktiv ist, zu einer gleichmäßigen formstabilen Aushärtung der Polyurethane auch schon bei Raumtemperatur führt und Probleme wie eine frühzeitige Hautbildung und eine verzögerte Reaktion im Kern des Produktes reduziert. Die erfindungsgemäßen Katalysatoren sollen temperaturstabil sein und auch zur Verwendung in lagerstabilen Einkomponentensystemen geeignet sein.

Diese Aufgabe wird erfindungsgemäß gelöst durch Ansprüche 1, 4 und 5.

R¹ und R⁴ sindvorzugsweise unabhängig voneinander C4-bisC9-Alkylen-gruppen, diebesonders bevorzugt linear sind. R² und R³ sind unabhängig voneinander bevorzugt lineare oder verzweigte C2- bis C4- Alkylengruppen, die z.B. 1,2-verknüpft, 1,3-verknüpft oder 1,4-verknüpft sind. Die Alkylen-Gruppen R¹ bis R⁴ sind zweibindig und müssen nicht α,ω-verknüpft (erstes / letztes C-Atom bezogen auf die längste Kette) sein, sondern können an einem oder zwei beliebigen C-Atomen der Alkylgruppen R¹ bis R⁴ in die erfindungsgemäße Verbindung eingebaut sein.

Lineare Alkylengruppen im Sinne der Erfindung sind z.B. folgende Alkylen-gruppen: CH₃-C.H-CH₂-C-H-CH₃ (1,3- verknüpfte Alkylengruppe) und CH₂-(CH₂)ₙC·H₂ (α,ω- verknüpfte Alkylengruppe). Verzweigte Alkylengruppen im Sinne der Erfindung sind z.B. folgende Alkylengruppen: CH₃-C·(CH₃)-CH₂-C·H-CH₃ (1,3-verknüpfte und Methylverzweigte Alkylengruppe) und ·CH₂-CH(CH₃)-(CH₂)ₙ-C·H₂ (α,ω-verknüpfte und Methyl verzweigte Alkylengruppe)

Weniger bevorzugt sind Bis(alkylamino)alkylether-Verbindungen, die zwei unsubstituierte oder alkylsübstitulerte Morpholin-Ringe und ausschließlich 1,2-verknüpfte Alkylen-Gruppen als R² und R³ Gruppe aufweisen.

Gegenstand der Erfindung sind weiterhin Polyurethan- und/oder Polyharnstoff-Zusammensetzungen enthaltend ein Polyurethan- und oder Polyharnstoff-Prepolymer oder zumindest eine der unten aufgeführten Komponenten zur Herstellung von Polyurethan- und/oder Polyharnstoff-Verbindungen und eine erfindungsgemäße Bis(alkylamino) alkylether-Verbindung als Katalysator. Wobei diese vorzugsweise zu 0,1 bis 6 Gew.%, besonders bevorzugt von 0,1 bis 2 Gew.%, in der Zusammensetzung enthalten ist.

Die erfindungsgemäßen Katalysatoren bzw. Zusammensetzungen werden bevorzugt zur Herstellung von Polyurethan- und/oder Polyharnstoff-Schaumstoffe mit einer Dichte von 10 bis 1200 kg/m³ , vorzugsweise von 10 bis 800 kg/m³ oder insbesondere von 10 bis 100 kg/m³ eingesetzt. Polyurethan-und/oder Polyharnstoff-Schaumstoffen mit Dichten von 10 bis 100 kg/m³ sind mikrozellulare Schäume, wie sie als Isolier- und Montageschäume eingesetzt werden. Polyurethan- und/oder Polyharnstoff-Schaumstoffe mit Dichten von 30 bis 800 kg/m³ sind Formschäume,

Im folgenden sollen Polyurethane, wie sie mit Hilfe. der erfindungsgemäßen Katalysatoren hergestellt werden können, beschrieben werden.

Zur Herstellung der erfindungsgemäßen Polyurethanschäume sind mehrere Bestandteile wesentlich. Dies sind vor allem aliphatische und/oder aromatische Polyisocyanate. Gebräuchliche Vertreter aliphatischer Polyisocyanate sind z.B. 1,6-Diisocyanatohexan, 3,5,5-Trimethyl-1-isocyanato-3-isocyanato-methylcyclohexan, 4,4'-Diisocyanato-dicyclohexylmethan, trimeres Hexandüsocyanat und 2,6-Diisocyanatohexansäuremethylester. Gebräuchliche aromatische Polyisocyanate sind 2,4-Diisocyanato-toluol, 1,5-Diisocyanato-naphthalin und 4,4'-Diisocyanatodiphenylmethan.

Die hydroxyhaltigen Polyolkomponenten, die mit dem Isocyanat reagieren, sind bevorzugt hydroxyhaltige Polyester oder Polyole. Die größte Bedeutung für die erfindungsgemäßen Katalysatoren haben Polyetherpolyole, wie etwa Polypropylenoxid oder Polyethylenoxid bzw. deren Copolymerisate. Diese können z.B. mit Hilfe di- oder mehrfunktioneller Alkohole als Startermoleküle hergestellt worden sein. Die Polyether-Komponente weist vorzugsweise ein Molekulargewicht zwischen 800 g/mol und 2000 g/mol und eine Funktionalität von 1,5 bis 3 auf.

Zur Herstellung der erfindungsgemäß eingesetzten Polyester werden di- und trifunktionelle Polyole mit Dicarbonsäuren bzw. deren Anhydride polykondensiert. Geeignete Polyole sind z.B. Ethylenglykol, 2-Propandiol,1,4-Butandiol, 1,6-Hexandiol, Glycerin, Trimethylolpropan, Pentaerythrit, Glycerin und Hexantriol. Geeignete Dicarbonsäuren bzw. Anhydride sind Bemsteinsäure, Adipinsäure, Phthalsäure und Isophthalsäure. Die Polyesterweisen vorzugsweise ein Molekulargewichtzwischen 300 und 3000 auf und eine verhältnismäßig hohe Hydroxyzahl bei einer verhältnismäßig niedrigen Säurezahl.

Hamstoffgruppen lassen sich In die erfindunggemäß hergestellten Polyurethane durch Einsatz von Wasser oder Diaminen einführen. Ethylendiamin, 1,2-Propylendiamin, Diaminocyclohexan oder Piperizin wirken z.B. als Kettenverlängerer oder Vernetzungsmittel. Polyurethan-Präpolymere mit Amin-Endgruppen sind reaktiver als solche mit Hydroxygruppe, so daß diese Polyurethane schneller aushärten. Ebenso können Verbindungen mit Polymercapto-Gruppen eingesetzt werden.

Im Rahmen der Erfindung können die Polyurethane nach unterschiedlichen Herstellungsverfahren hergestellt sein. Nach dem One-Shot-Verfahren werden Polyurethane vorzugsweise in Abwesenheit von Lösungsmitteln in Gegenwart sämtlicher Reaktionskomponenten hergestellt. Das Verfahren setzt eine annähernd vergleichbare Reaktionsgeschwindigkeit der verschiedenen H-aktiven Komponenten und der Polyisocyanate voraus.

Man unterscheidet hiervon das Präpolymerverfahren. Polyurethan-Präpolymere sind Zwischenstufen der Isocyanat-Polyaddition.-Man-unterscheidet.NCO-Präpolymere.mit.endständigen.NCO-.Gruppen und Hydroxy-Präpolymere. Die NCO-Präpolymere haben eine besondere Bedeutung, da sie mit einer Vielzahl von aktiven Wasserstoff enthaltenden Verbindungen ausgehärtet werden können. Sie werden durch Umsetzung von Di- bzw. Polyhydroxyverbindungen mit einem molaren Überschuß an Di- bzw. Polyisocyanat erhalten. Diese Gemische können noch einen beträchtlichen Prozentsatz des monomeren Isocyanates enthalten. Die erfindungsgemäßen Katalysatoren werden bevorzugt in oder für Präpolymere eingesetzt.

Das Polyurethan-Präpolymer hat vorzugsweise eine Funktionalität von etwa 2 bis 4 und ein Molekulargewicht von etwa 100 bis 3000.

Zur Herstellung von Polyurethanen können Einkomponentensysteme oder Zweikomponentensysteme eingesetzt werden. Man unterscheidet physikalisch trocknende Einkomponentensysteme und durch Einwirkung von Luftfeuchtigkeit trocknende Einkomponentensysteme. Letztere enthalten vorzugsweise verhältnismäßig niedermolekulare Präpolymere (200 - 2000 g/mol) mit endständigen NCO-Gruppen. Hier ist die Aushärtungsgeschwindigkeit abhängig von der jeweiligen Feuchte der Luft und der Temperatur. Ein wichtiges Erfordernis solcher Einkomponentensysteme ist die Lagerstabilität. Die erfindungsgemäßen Katalysatoren werden bevorzugt In/für feuchtigkeltshärtende Einkomponentensysteme eingesetzt, wobei es ein Verdienst der erfindungsgemäßen Katalysatoren ist, vor allem im Inneren des Polyurethan-Kunsstoffes für eine ausreichend schnelle Härtung , d.h. einen raschen Festigkeitsaufbau, auch schon bei niedrigeren Temperaturen wie etwa Raumtemperatur und normaler Umgebungsfeuchte zu sorgen.

Zweikomponentensysteme bestehen meist aus einer Polyhydroxidkomponente die auch schon durch ein Diisocyanat zu einem Präpolymer mit endständigen OH-Gruppen verlängert sein kann. -als Hauptkomponente und einem Isocyanat-Addukt als Vernetzer

Die erfindungsgemäßen Katalysatoren können, zumindest bezogen auf den gesamten Herstellungsprozeß, in Kombination mit anderen Polyurethan-Katalysatoren eingesetzt werden.

Im Rahmen der Erfindung kann auch ein von außen zugesetztes inertes Treibmittel, z.B. ein Gas oder ein gasbildender Stoff angewandt werden. Beispielsweise können niedrigsiedende Kohlenwasserstoffe wie Propan, Butan, Methylenchlorid und dgl. verwendet werden.

Ferner können übliche Formulierungszusätze verwendet werden., beispielsweise Weichmacher, Trocknungsmittel, Füllstoffe, latente Härter, Haftungsverbesserer, Schaumstabilisatoren, die auch als Silikonöle oder Emulgatoren bekannt sind. Dies sind z.B. Polysiloxan-polyether-copolymerisate unterschiedlichsten Aufbaus. Weitere mögliche Zusätze sind Hydrolysestabilisatoren, Oxydationsstabilisatoren, UV-Stabilisatoren, Flammschutzmittel oder auch Farbstoffe, vorzugsweise in Form von Farbpasten.

### Beispiele

### Beispiel 1 : Herstellung von 2,2'-Bis(hexamethyl-imino)-diethylether

Das oben genannte Produkt besitzt die Strukturformel:

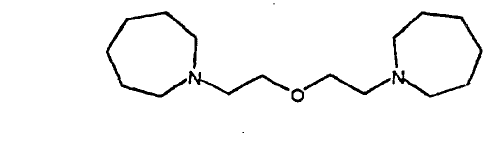

Es wurde hergestellt aus

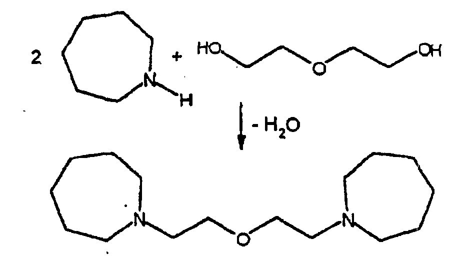

Die-Reaktion-wurde-wie-folgt-durchgeführt-Die-Reaktion-wurde in einem Pilotanlage mit einem Katalysatorfüllvolumen von 100 cm³ duchgeführt. Es wurde ein Kupferchromit enthaltender Katalysator verwendet. Die übrigen Reationsbedingungen sind in Tabelle 1 zusammengestellt:

**Tabelle 1**

| Reaktionsbedingungen der Synthese | |
|---|---|
| Temperatur | 180°C |
| Druck | 10 bar |
| Zuflußrate (flüssig) | 24 ml/h |
| Gaszufluß (H₂) | 12 l/h |

Eine so erhaltene Reaktionsmischung von 480 g wurde per Gäschromatographie analysiert: Danach wurde die Mischung zunächst unter Normaldruck und danach im Vakkuum destillativ gereinigt. Ebenso lassen sich erfindungsgemäße Bis(alkylamino)alkylether-Verbindungen herstellen, die mehrere Aklyloxy-Gruppen und für R¹ bzw. R⁴ z.B. C7- bis C9- Alkylengruppen aufweisen.

### Beispiel 2 : Herstellung von Katalysator C

Der in den Beispielen 3 bis 5 näher beschrieben Katalysator C hat die folgende Strukturformel: 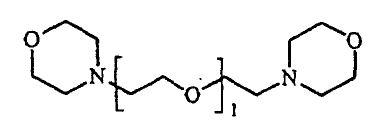 l = im Mittel ca. 3,3 (hauptsächlich Gemisch aus l= 3 und l= 4)

Er wurde wie folgt hergestellt 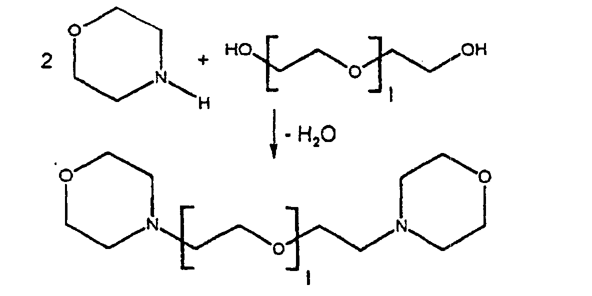 l = im Mittel ca. 3,3 (hauptsächlich Gemisch aus l = 3 und l = 4)

Die Reaktion wurde im Rohrreaktor bei 210°C und 40 bar durchgeführt-. Als Katalysator wurde eine Mischung mit 25% CuO und 1% Cr₂O₃ eingesetzt. Es wurde eine Mischung mit 20 bis 40% Produkt erhalten. Diese Mischung wurde durch Vakuumdestillation gereinigt.

### Beispiel 3 : Formulierung zur Herstellung eines feuchtigkeitshärtenden 1-Komponenten Schaums A

| | |
|---|---|
| Prepolymer | 25 g |
| Silicon PC STAB EP 05 | 0,25 g |
| Katalysator | siehe unten |

Die Komponenten wurden im Labormaßstab bei ca. 1500 U/min über eine Zeit von 20 s eingerührt und danach auf einen Pappträger in einer Schichtdicke von ca. 1-2 mm aufgetragen.

Die Versuche wurden bei einer Luftfeuchtigkeit von ca. 50 % bei Raumtemperatur durchgeführt. Die Aushärtung erfolgt in erster Linie durch die Luftfeuchtigkeit, aber auch durch das von dem Pappträger zur Verfügung gestellte Wasser. Auch in der technischen Anwendung werden häufig freies Wasser enthaltende Kontaktmaterialien, wie z.B. Holz eingesetzt, so daß der Pappträger als Kontaktmaterial Vergleichscharakter hat. Um eine Vergleichbarkeit der Aktivität der Katalysatoren zu erreichen wurde die Einsatzmenge so gewählt, daß ein gleicher Amingehalt in allen Vergleichsmessungen gegeben war.

Es wurden folgende erfindungsgemäße Katalysatoren mit varrierendem l eingesetzt : 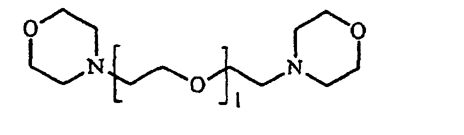
Katalysator A : l=4
Katalysator B : l= 3
Katalysator C l = ca. 3.3 (Gemisch aus l = 3 und l = 4)

Als Referenz wurde Dimorpholinodiethylether (DMDEE) eingesetzt. Die Ergebnisse sind in der folgenden Tabelle 1 vergleichend dargestellt.

**Tabelle 2**

| ü | Katalysator A | Katalystor B | Katalystor C | DMDEE |
|---|---|---|---|---|
| Einsatzmenge [g] | 1,16 | 1,02 | 1,02 | 0,75 |
| Beginn der Hautbildung [min.] | 6. | 7 | 5 | 3 |
| Klebfreizeit [min.] | 9 | 9 | 7 | 5 |
| innere Aushärtung nach 15 min | keine | beginnend | beginnend | fast vollständig |
| innere Aushärtung nach 30 min | beginnend | teilweise | fast vollständig | fast vollständig |
| innere Aushärtung nach 45 min | teilweise | fast vollständig | vollständig | vollständig |

Die Beobachtungen zeigen, daß die eingesetzten erfindungsgemäßen Katalysatoren die Hautbildung verzögem ohne gleichzeitig die Endaushärtung zu verlangsamen. Insbesondere Katalysator C führt zu Beginn der Reaktion zu einer Verlangsamung. Die Endaushärtung wird jedoch zum gleichen Zeitpunkt wie beim Standardkatalysator DMDEE erreicht.

### Beispiel 4 Formulierung zur Herstellung eines feuchtigkeitshärtenden 1-Komponenten Schaums B:

| | |
|---|---|
| Prepolymer | 75 g |
| Silicon PC STAB EP 05 | 0,75 g |
| Katalysator | siehe unten |

Die Komponenten wurden im Labormaßstab bei ca 1500 U/min über eine Zeit von 20 s eingerührt und danach auf ein Glasplatte in einer Schichtdicke von ca. 1 cm aufgetragen.

Die Versuche wurden bei einer Luftfeuchtigkeit von ca. 50 % bei Raumtemperatur durchgeführt. Die Aushärtung erfolgt ausschließlich durch die Luftfeuchtigkeit. Die Katalysatormenge wurde so eingestellt, daß eine Vergleichbarkeit der Klebfreizeit erreicht wurde.

Als Vergleichsmaterial wurde der für diese Anwendung industriell eingesetzte Standardkatalysator DMDEE benutzt.

**Tabelle 3**

| | Katalysator C (siehe Beispiel 2 ) | DMDEE |
|---|---|---|
| Einsatzmenge [g] | 3,06 | 1,5 |
| Klebfreizeit [min ] | 4 | 4 |

Aushärtung nach 18 h:
- Katalysator C:: Die Probe war nach 18 h vollständig ausgehärtet. Es war nur eine leichte Aufwölbung der Oberfläche zu beobachten. Die Oberfläche war geschlossen und ohne Risse oder andere Defekte
- DMDEE:: Die Probe war nach 18 h noch nicht vollständig ausgehärtet. Im Inneren der Probe war noch ein Teil der hochviskosen Mischung vorhanden. Die Oberfläche war stark aufgewölbt und geschlossen zeigte jedoch kleine Risse und Defekte. Beim Öffnen der Oberfläche zeigte sich eine erheblicher Innendruck.

Die Beobachtungen zeigen, daß bei größeren Schichtdicken und ausschließlicher Aushärtung aufgrunder Luftfeuchtigkeit der erfindungsgemäße Katalysator Katalysator C bei gleicher Klebfreizeit im Vergleich zum Standard zu einer deutlich schnelleren Endaushärtung führt. Außerdem wurde die Tendenz zur Nachreaktion, sichtbar durch Risse, Defekte und das Aufwölben der Schaumoberfläche durch den Einsatz von Katalysator C deutlich reduziert.

### Beispiel 5 : Formulierung zur Herstellung eines feuchtigkeitshärtenden 1-Komponenten Dosenschaums.

Unter Verwendung einer industriell eingesetzten Formulierung aus Prepolymer Silikon und Treibmittel wurden unter Einsatz von zwei der erfindungsgemäßen Katalysatoren handelsübliche Montageschaumdosen hergestellt. Diese wurden im vergleichenden Test mit den handelsüblichen Dosen basierend auf dergleichen Basisformulierung unter Einsatz der industriellen Standardkatalysatoren DMDEE verprobt.

**Tabelle 4**

| | DMDEE (Vergleich) | Katalysator A (siehe Beisp. 3) | Katalysator C (siehe Beisp. 2) |
|---|---|---|---|
| Einsatzmenge [Gew.%] | 1,2 | 2,5 | 2,4 |
| Schaumausbeute [rel.] | 1 | ca. 0,90 | ca. 0,95 |
| Hautbildung | Spontan | verzögert | spontan |

innere Aushärtung:
- DMDEE:: Die innere Aushärtung verläuft langsam, es tritt eine relativ starke Nachreaktion auf, was sich in einem Nachdrücken des Schaums bemerkbar macht.
- Katalysator A: Es findet eine verzögerte Aushärtung statt. Ein Nachdrücken des Schaums wird nur In deutlich abgeschwächter Form beobachtet.
- Katalysator C: Die innere Aushärtung verläuft gegenüber dem Vergleich beschleunigt. Die Neigung zu einer Nach reaktion ist gegenüber dem industriellen Standard (DMDEE) deutlich reduziert.

Die Lagerstabilität wird durch die neuen erfindungsgemäßen Katalysatoren im Vergleich zu den bisher eingesetzten Standardkatalysatoren, wie z.B. DMDEE nicht beeinflußt.

Die Versuche 3 bis 5 belegen, daß die eingesetzten erfindungsgemäßen Katalysatoren in einer industriell eingesetzten Formulierung zur deutlichen Verbesserungen in Bezug auf das Verhalten von Hautbildung und Endaushärtung ( innere Druckbildung ) im Vergleich zum industriellen Standardkatalysator DMDEE führen.

## Patentansprüche

1. Polyurethan- und/oder Polyharnstoff-Zusammensetzung enthaltend
(A) ein Polyurethan- und / oder Polyharnstoff-Prepolymer oder eine Polyurethan- und / oder PolyharnstoffVerbindung und
(B) eine Bis(alkylamino)alkylether-Verbindung der Formel 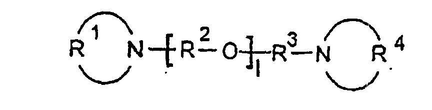
worin,
R¹ und R⁴ unabhängig voneinander eine lineare oder verzweigte C4-bis C9- Alkylengruppe sind,
R² und R³ unabhängig voneinander eine lineare oder verzweigte C2-bis C6- Alkylengruppe sind, wobei R² für jedes 1 unterschiedlich sein kann, und
^{l} 3,4,5 oder 6 ist.

2. Polyurethan- und/oder Polyharnstoff-Zusammensetzung enthaltend Bis(alkylamino)alkylether-Verbindungen gemäß Anspruch 1, worin
R¹ und R⁴ unabhängig voneinander eine lineare C4- bis C9- Alkylengruppe sind.

3. Polyurethan- und/oder Polyharnstoff-Zusammensetzung gemäß einem der vorhergehenden Ansprüche, enthaltend 0,1 bis 6 Gew.%, bezogen auf die Gesamtzusammensetzung, die Bis(alkylamino)-alkylether-Verbindung (B).

4. Verwendung einer Bis(alkylamino)alkylether-Verbindung der Formel 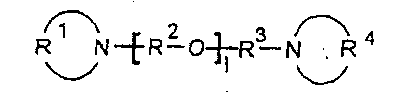 worin.
R¹ und R⁴ unabhängig voneinander eine lineare oder verzweigte C4-bis C9- Alkylengruppe und / oder eine zweibindige Di-(C1-bis C4-alkyl)ether-Gruppe sind,
R² und R³ unabhängig voneinander eine lineare oder verzweigte C2-bis C6- Alkylengruppe sind, wobei R² für jedes 1 unterschiedlich sein kann, und
I 3,4,5 oder 6 ist.
zur-Herstellung-von-Polyurethan- und/oder-Polyharnstoff-Schaumstoffen mit einer Dichte von 10 bis 800 kg/m³, insbesondere mit einer Dichte von 10 bis 100 kg/m³.

5. Bis(alkylamino)alkylether-Verbindungen 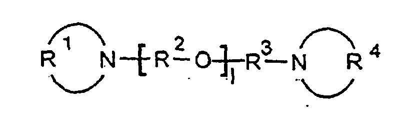 worin,
R¹ eine lineare oder verzweigte C4- bis C9- Alkylengruppe und/oder eine zweibindige Di-(C1- bis C4-alkyl)ether-Gruppe ist,
R4 eine α,ω- verknüpfte lineare C7- bis C9- Alkylengruppe ist oder eine verzweigte C4- bis C9- Alkylengruppe ist oder eine nicht α,ω- verknüpfte lineare C4- bis C9- Alkylengruppe ist oder
eine zwelbindige Di-(C1-bis C4-alkyl)-ether-Gruppe ist, wobei zumindest eine der Alkylgruppen der Bisalkylether-gruppe nicht 1,2- verknüpft ist, und
R² und R³ unabhängig voneinander eine lineare oder verzweigte C2-bis C6- Alkylengruppe sind, wobei R² für jedes l unterschiedlich sein kann, und
l eine ganze Zahl von 3 bis 6 ist.

## Claims

1. Polyurethane and/or polyurea composition containing
(A) a polyurethane and/or polyurea prepolymer or a polyurethane and/or polyurea compound and
(B) a bis(alkylamino)alkylether compound of the formula 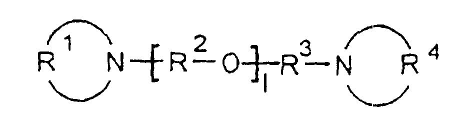
wherein
R¹ and R⁴ are, independent of one another, a linear or branched C4- to C9-alkylene group,
R² and R³ are, independent of one another, a linear or branched C2- to C6-alkylene group, wherein R² can be different for each 1, and
1 is 3, 4, 5 or 6.

2. Polyurethane and/or polyurea composition containing bis(alkylamino)alkylether compounds according to claim 1, wherein
R¹ and R⁴ are, independent of one another, a linear C4- to C9-alkylene group.

3. Polyurethane and/or polyurea composition according to one of the preceding claims, containing 0.1 to 6% by weight of the bis(alkylamino)alkylether compound (B), relative to the total composition.

4. Use of a bis(alkylamino)alkylether compound of the formula 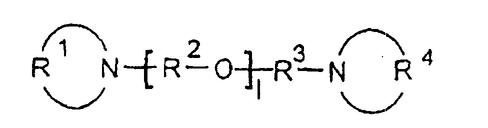 wherein
R¹ and R⁴ are, independent of one another, a linear or branched C4- to C9-alkylene group and/or a divalent di-(C1- to C4-alkyl)ether group,
R² and R³ are, independent of one another, a linear or branched C2- to C6- alkylene group, wherein R² can be different for each l, and
l is 3, 4, 5 or 6.
for the production of polyurethane and/or polyurea foams with a density of 10 to 800 kg/m³, particularly with a density of 10 to 100 kg/m³.

5. Bis(alkylamino)alkylether compounds 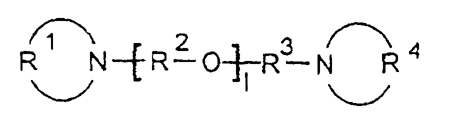 wherein
R¹ is a linear or branched C4- to C9-alkylene group and/or a divalent di-(C1- to C4-alkyl) ether group,
R⁴ is an α, ω-linked linear C7-to C9-alkylene group, or
is a branched C4-to C9-alkylene group, or
is a non-α, ω-linked linear C4- to C9-alkylene group, or
is a divalent di-(C1- to C4-alkyl) ether group, wherein at least one of the alkyl groups of the bis-alkylether group is not 1,2-linked, and
R² and R³ are, independent of one another, a linear or branched C2- to C6-alkylene group, wherein R² can be different for each 1, and
l is an integer from 3 to 6.

## Revendications

1. Composition de polyuréthanne et/ou polyurée contenant
(A) un prépolymère de polyuréthanne et/ou polyurée ou un composé de polyuréthanne et/ou polyurée et
(B) un composé bis(alkylamino)alkyléther de formule 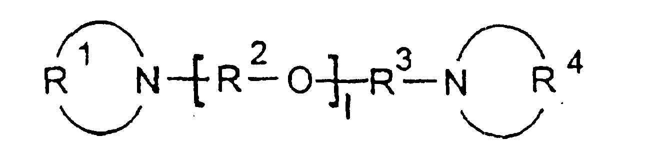
dans laquelle,
R¹ et R⁴ représentent indépendamment l'un de l'autre un groupe alkylène en C₄ en C₉ linéaire ou ramifié,
R² et R³ représentent indépendamment l'un de l'autre un groupe alkylène en C₂ en C₆ linéaire ou ramifié, R² pouvant être différent pour chaque l, et
l vaut 3, 4, 5 ou 6.

2. Composition de polyuréthanne et/ou polyurée contenant le composé bis(alkylamino)alkyléther selon la revendication 1, dans laquelle
R¹ et R⁴ représentent indépendamment l'un de l'autre un groupe alkylène en C₄ en C₉ linéaire.

3. Composition de polyuréthanne et/ou polyurée selon l'une quelconque des revendications précédentes, contenant le composé bis(alkylamino)alkyléther (B) à raison de 0,1 à 6% en poids, par rapport à la composition entière.

4. Utilisation d'un composé bis(alkylamino)alkyléther de formule 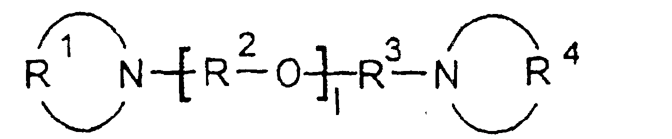 dans laquelle,
R¹ et R⁴ représentent indépendamment l'un de l'autre un groupe alkylène en C₄ en C₉ linéaire ou ramifié et/ou un groupe di-(alkyle en Ci en C₄)-éther à deux liaisons,
R² et R³ représentent indépendamment l'un de l'autre un groupe alkylène en C₂ en C₆ linéaire ou ramifié, R² pouvant être différent pour chaque l, et
l vaut 3,4,5 ou 6
en vue de préparer des mousses de polyuréthanne et/ou polyurée ayant une masse volumique de 10 à 800 kg/m³, en particulier de 10 à 100 kg/m³.

5. Composés bis(alkylamino)alkyléther 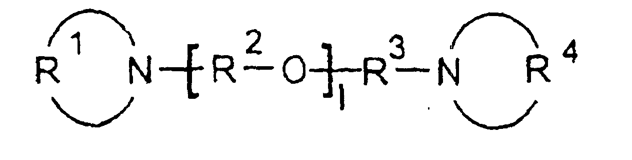 dans lesquels
R¹ représente un groupe alkylène en C₄ en C₉ linéaire ou ramifié et/ou un groupe di-(alkyle en C₁ en C₄)-éther à deux liaisons,
R⁴ représente un groupe alkylène en C₇ en C₉ linéaire lié en α, ω ou
un groupe alkylène en C₄ en C₉ ramifié ou un groupe alkylène en C₄ en C₉ linéaire non lié en α, ω ou
un groupe di- (alkyle en C₁ en C₄) -éther à deux liaisons, où au moins un des groupes alkyle du groupe bisalkyléther n'est pas lié en 1,2, et
R² et R³ représentent indépendamment l'un de l'autre un groupe alkylène en C₂ en C₆ linéaire ou ramifié, R² pouvant être différent pour chaque l, et
l représente un nombre entier de 3 à 6.
